# EUROPEAN PATENT SPECIFICATION

(11) **EP 1 001 924 B1**
(45) Date of publication and mention of the grant of the patent: **23.04.2003**
(21) Application number: 98933874.4
(22) Date of filing: 27.07.1998
(51) Int. Cl.: C07C 50/36, C07C 69/95, A61K 31/12, A61K 31/235, A61K 41/00

(54) **THE USE OF 1,3,4,6-TETRAHYDROXY-HELIANTHRONE AND ITS DERIVATIVES IN PHOTODYNAMIC THERAPY AND CERTAIN SUCH NOVEL DERIVATIVES**
VERWENDUNG V0N 1,3,4,6-TETRAHYDROXY-HELIANTHRON UND DERIVATEN IN DER PHOTODYNAMISCHEN THERAPIE UND DERIVATE
UTILISATION DE 1,3,4,6-TETRAHYDROXY-HELIANTHRONE ET DE SES DERIVES DANS LE TRAITEMENT PHOTODYNAMIQUE ET CERTAINS NOUVEAUX DERIVES

(30) Priority: 31.07.1997 IL 12144097
(43) Date of publication of application: 24.05.2000
(73) Proprietor: YEDA RESEARCH & DEVELOPMENT COMPANY, LTD., 76100 Rehovot (IL); NEW YORK UNIVERSITY, New York, NY 10012 (US)
(72) Inventor: MAZUR, Yehuda, 62917 Tel-Aviv (IL); LAVIE, Gad, 76472 Rehovot (IL)
(74) Representative: Vaillant, Jeanne
(86) International application number: PCT/IL98/00346
(87) International publication number: WO 99/006347

(56) References cited:
- EP-A- 0 390 181
- WO-A-94/14956
- WO-A-94/27952
- WO-A-96/07731
- RODEWALD G ET AL: "Synthesis of hypericin and related meso-naphthodianthrones by alkaline dimerization of hydroxyanthraquinones" ANGEW. CHEM. (ANCEAD);77; VOL.89 (1); PP.56-7, XP002084088 Univ. Bonn;Inst. Org. Chem. Biochem.; Bonn; Ger. cited in the application
- WEINER L ET AL: "EPR studies of hypericin. Photogeneration of free radicals and superoxide" J. CHEM. SOC., PERKIN TRANS. 2 (JCPKBH,03009580);92; (9); PP.1439-42, XP000500106 Weizmann Inst. Sci.;Dep. Chem. Phys.; Rehovot; 76 100; Israel (IL)

## Description

The present invention concerns the novel therapeutic use of 1,3,4,6-tetrahydroxy-helianthrone (hereinafter "*THe*") and substituted derivatives thereof as photoactivators for photodynamic (PD) therapy. The present invention also provides novel substituted derivatives of THe especially 10,13-dimethyl-1,3,4,6-tetrahydroxy-helianthrone (hereinafter "*DTHe*").

### BACKGROUND OF THE INVENTION

The incorporation of photosensitizing molecules into therapy of dispersed as well as solid tumors is becoming increasingly prevalent. Solid tumors are usually sensitized with porphyrin derivatives and irradiated with light for activation⁽¹⁾. Hairy cell leukemia and mycosis fungoides have also been treated with 8-methoxypsoralen and U.V.A. (PUVA)⁽²⁾. Criteria for selecting photoactivators for photodynamic (PD) therapy have remained empirical using primarily porphyrin derivatives, due to speculation that low photodynamic activity can be compensated by more intense doses of light irradiation. PD therapy, however, is now progressing to the use of novel compounds. Our preference is for agents whose mode of action combines high quantum yields of singlet oxygen with additional potentiating properties: secondary inhibitory activities of cell proliferation, signal transduction pathways and higher affinity to tumor cell membranes.

Hypericin (hereinafter "*HY*") is a polycyclic dianthraquinone which is known as a potent photodynamic agent. In the presence of light, HY generates singlet oxygen⁽³⁾, free radicals⁽⁴⁾, semiquinones⁽⁵⁾ and provides light-induced pH drop of its surroundings. The biological and virucidal activities of HY have been thoroughly investigated (for review see Ref. 6), and it is also known to act as a protein kinase C (PKC) inhibitor, particularly when PKC is translocated to the cell membrane following cell activation⁽⁷⁾. HY also exhibits inhibitory activity to epidermal growth factor (EGF) receptor tyrosine kinase and MAP kinase⁽⁷⁾. The molecule was shown to be cytotoxic to fibroblasts and to mammary carcinoma cells *in vitro.*

WO 94/27,952 describes hypercin compounds and their use as antiviral agents.

WO 94/14,956 describes a composition for inactivating viruses and destroying tumor cells comprising a photosensitizing chemical, an energy donating chemical. The photosensitizing chemical is selected from several groups among them hypercin.

Weiner et al. "EPR studies of hypericin. Photogeneration of free radicals and superoxide" J. CHEM. SOC., PERKIN TRANS. 2 (JCPKBH, 03009580);92; (9); PP. 1439-42 describe hypericin as an antiviral agent: The sodium salt of hypericin (HY-Na) and its lysine salt (HY-Lis) are described.

Rodewald et al. "Synthesis of hypericin and related mesonaphthodianthrones by alkaline dimerization of hydroxyanthraquinones" ANGEW. CHEM. (ANCEAD);77; VOL.89 (1); PP.46-7 describe the synthesis of hypercin and some hypercin derivatives.

Hypericin may cause severe side effects such as prolonged post-treatment sensitivity to light, a condition medically known as Hypericism. The mechanisms by which Hypericin elicits its cytotoxic effect, are via an apoptotic pathway at low photodynamic stress levels and at higher photodynamic stress level via necrosis. It would be desirable to provide additional photosensitizing agents which can elicit their cytotoxic effect with higher efficiency in comparison with existing agents and, potentially, with lower and less severe side effects.

### SUMMARY OF THE INVENTION

The present invention is based on the surprising finding that although HY is a more potent photosensitizer than DTHe, the latter exhibited stronger phototoxic properties in HL-60 and K-562 cells. DTHe induced cell death at an LD₅₀ dose 3-5 fold lower than the LD₅₀ dose of HY under identical lighting conditions as determined by the MTT assay.

DTHe was found to protect L-cells from cell death induced by TNF-α, the tumor necrosis factor. TNF-α is a cytokine that participates in inflammations inducing the death of numerous cells by apoptosis and is believed to contribute to tissue damage during inflammatory reactions. DTHe was also effective in inhibiting human natural killer (NK)cell mediated cytotoxicity.

The similarity in the lytic mechanisms of target cell killing by NK cells and by T-cells suggest that DTHe or compounds of the present invention are also effective in preventing target cell toxicity by T-cells in a manner similar to NK cells. Thus, the compounds of the present invention are anticipated to be potent inhibitors of T-cell mediated diseases.

There is hereby described the use of 1,3,4,6-tetrahydroxy-helianthrone compounds of the formula (I) 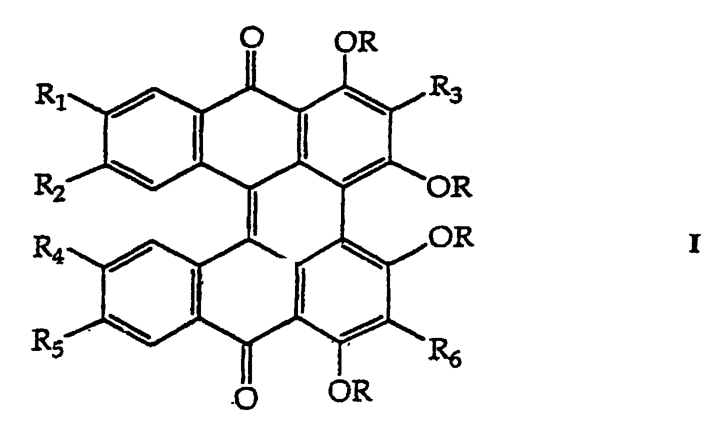 in which R is hydrogen or methyl and each of R₁, R₂, R₃, R₄, R₅ and R₆ is independently selected from H, OH, Cl, Br, methyl, and methoxycarbonyl, in the manufacture of pharmaceutical compositions for use in photodynamic therapy, to elicit destruction of tumors in conjunction with light.

Preferred compounds of formula (I) are those wherein each of R₂ and R₄ is methyl or methoxycarbonyl i.e. the 10,13-dimethyl and 10,13-di-(methoxycarbonyl)-1,3,4,6-tebrahydroxy-helianthrone derivatives, and R, R₁, R₃, R₅ and R₆ are each hydrogen. These compounds were, surprisingly, found to be retained better within tumor cells, in comparison with compounds in which R₂ and R₄ are hydrogens.

The claims concern the use of the compounds of formula (I) in which R is hydrogen or methyl, each of R₁, R₂, R₃, R₄, R₅ and R₆ is independently selected from H, OH, Cl, Br, methyl and methoxycarbonyl, and particularly the use of the compounds of formula I in which R is hydrogen, R₂ and R₄ are the same and are each methyl or methoxycarbonyl and R₁, R₃, R₅ and R₆ are each H, in the manufacture of pharmaceutical compositions for use in photo dynamic therapy of tumors, to elicit destruction of tumors in conjunction of light.

In accordance with another aspect, there is hereby described a method of photodynamic therapy of tumors consisting of injecting to a patient an appropriate amount of a compound of formula (I) above, followed by local irradiation.

The compounds of formula (I) as hereby described are thus suitable for use in the PDT of various types of both malignant and benign tumors, including, e.g., squamous cell carcinoma, basal cell carcinoma, melanoma, kaposi sarcoma, breast carcinoma, hemangioma, meningioma, astrocytoma, neuroblastoma, carcinoma of the pancreas, colorectal carcinoma, colon carcinoma, transitional cell carinoma of the bladder and carcinoma of the larynx, and benign tumours, e.g.,verruca vulgaris, condyloma and fibroma.

The pharmaceutical compositions of the invention will be administered to the patient by standard procedures used in PDT. The amount of compound to be administered and the route of administration will be determined according to the kind of tumour, stage of the disease, age and health conditions of the patient, but will be much lower that currently used dosage of Photofrin II of about 20-40 mg/kg body weight. The preferable routes of administration are intravenous or direct injection into the solid tumor of the aqueous solution of the active compound comprising conventional pharmaceutically acceptable carriers and additives, and topical treatment of the skin tumours with suitable topical compositions.

The method of photodynamic therapy of cancer according to the present description, comprises administering to a patient afflicted with a solid tumour cancer, a pharmaceutical composition comprising a compound of Formula (I), and then irradiating the tumour site with strong light sources at 450-600 nm, preferably at 490 nm.

In accordance with yet another aspect, there are described novel compounds of formula (I) above in which at least one of R,R₁, R₂, R₃, R₄, R₅ and R₆ is other than hydrogen.

The compound claims concern the compounds of formula (I) in which R is hydrogen or methyl, each of R₁, R₂, R₃, R₄, R₅ and R₆ is independently selected from H, OH, Cl, Br, methyl and methoxycarbonyl wherein at least one of R and R₁ to R₆ is other than hydrogen, and particularly those wherein R₂ and R₄ are the same and are each methyl or methoxycarbonyl and R₁, R₃, R₅ and R₆ are each H.

The compounds of formula (I) in which R₂ and R₄ are methyl can be prepared by the method described in US Patent 5,120,412 using as a starting material a 1,3-dihydroxy-6-(lower alkyl)-anthraquinone of the formula (II) 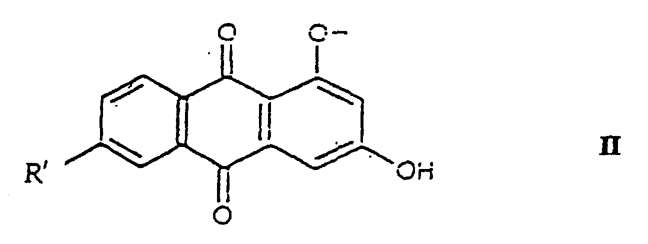 in which R' is methyl. Compound II is reduced to the corresponding anthrone of the formula (III) 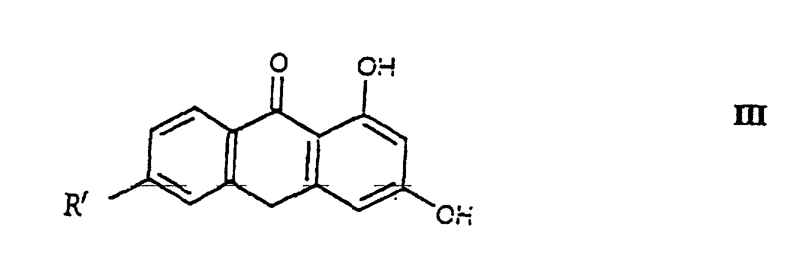 in which R' is as defined above and compound III is condensed to obtain desired compounds of formula (I) in which R is methyl.

Other compounds of formula (I) can be prepared in an analogous manner using appropriately substituted 1,3-dihydroxy-anthraquinones.

The compounds of formula (1) in which R₂ and R₄ are each methoxycarbonyl can be prepared from the diacetyl derivatives of the compound of formula (II) above in which R' is methyl by oxidation with CrO₃ to form the compound of the formula (IV) 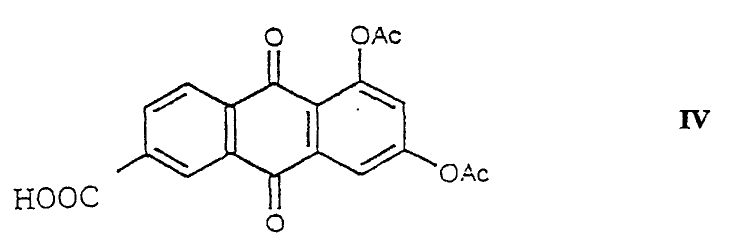 which is then dimerized by the method of Spitzner (*Angew. chem. Int. Ed*., **16**, 46 (1977)) to form a compound of formula (I) in which R is carboxy which is then esterified with methanol to obtain the desired product of formula (I) in which R is methoxycarbonyl.

### BRIEF DESCRIPTION OF THE DRAWINGS:

**Figs. 1A, 1B -**the effects of light dose and reagent concentrations on HL-60 cell viability in an MIT assay.
**Figs. 2A, 2B -**analysis of the mechanisms of cell death induced by DTHe and by HY on HL 60 cells, by microscopy.
**Figs. 3A, 3B -**microscopic analysis of the mechanisms of cell death induced by DTHe and by HY on K-562 cells.
**Fig. 4 -**shows the percentage of HL-60 cells featuring normal, apoptotic or necrotic morphology at different periods after administration of 0.65 M THe and light irradiation.
**Fig.5 -** protection of L cells from TNF-α induced cell death by DTHe.
**Fig.6 -** effect of DTHe on Nk cell activity against K-562 cells.

### PREFERRED EMBODIMENTS OF THE INVENTION

The invention will be described in more detail in the following non-limiting examples with reference to the accompanying drawings:

### PREPARATIVE EXAMPLES

### Example A

1,3-dihydroxy-6-methyl-anthraquinone (II R'=CH₃) (380 mg) was dissolved in 45 ml glacial acetic acid, heated to boiling and then treated dropwise with a solution of SnCl₂.2H₂O (9.6 g) in conc. HCl (24 ml) under stirring at 90° for 2 hours. The reaction mixture was then refluxed for an additional hour. The acetic acid was removed under reduced pressure and the residue treated with 200 ml of water. The formed precipitate was filtered and dried to give 300 mg of the anthrone (III R'=CH₃) which, without purification, was dissolved in a mixture of 9.45 ml of pyridine and 0.94 ml of piperidine. The resulting solution was treated with 940 mg of pyridine N-oxide and 0.05 g of ferrous sulphate heptahydrate and then refluxed for 1 hour at 100°. The reaction mixture was concentrated under vacuum and the solid product obtained was dissolved in acetone, then filtered, and the acetone solution evaporated to dryness. The residue was chromatographed on a silica gel column. The fraction eluted with a mixture of ethyl acetate-methanol (85:15) gave a red solution which was evaporated to dryness to give 60 mg (20% yield) of the 10,13-dimethyl-1,3,4,6-tetrahydroxy-helianthrone (I,R= CH₃).
NMR δ (CD₃CN): 2.13 (6H s 10,13-CH₃), 6.33 (2H s 2,5-H), 7.33 (2H d J=8Hz 9,14-H), 7.66 (2H s 11,12-H), 8.29 (2H d J-8Hz 8,15-H), 16.14 (1H s 3 or 4-H).

### Example B

1,3-dihydroxy-6-methyl-anthraquinone (II,R'=CH₃) (100 mg) was dissolved in pyridine (0.75 ml), treated with acetic anhydride (3.8 ml) and then refluxed for 3 hours. The reaction mixture was cooled to 0°C in an ice bath and the formed precipitate was filtered, washed with water and crystallized from ethyl acetate and hexane to give the 1,3-diacetoxy-6-methyl anthraquinone (80 mg).
NMR δ (CDCl₃): 2.34 (3H s 6-CH₃), 2.47 (3H s OAc), 2.50 (3H s OAc), 7.24 (1 H s 2-H), 7.56 (1 H dd J=8,0.6 Hz 7-H), 7,97 (1H d J=2.4 Hz 4-H), 8.03 (1H br 5-H), 8.09 (1H d J=8 Hz 8-H).

### Example C

1,3-diacetoxy-6-methyl-anthraquinone (20 mg) (obtained in Example B) was dissolved in a 1:1 mixture of acetic anhydride and acetic acid (16.6 ml) at 50°C and added dropwise to a solution of CrO₃ (135 mg) in aqueous 40% acetic acid (2.7 ml). The reaction mixture was then stirred for 3 hours, cooled and poured into 200 ml of water. After being left for 2 hours the solution was extracted with ethyl acetate washed with water and then extracted twice with saturated sodium bicarbonate solution. The aqueous phase was washed twice with methylene chloride, acidified with dilute HCl and extracted with ethyl acetate. The organic extract was evaporated to dryness yielding 18 mg of 1,3-diacetoxy-6-carboxy-anthraquinone (IV). UV-vis (EtOH) λₘₐₓ353, 483, 530sh, 564 nm ( ε 22000, 30000, 20000, 15000)
NMR δ (CD₃OD): 6.50 (1H s 2-H), 7.17 (1H s 5-H), 8.50 (2H dd J=34, 6 Hz 7,8-H), 8.77 (1H br 1-H).

### Example D

1,3-Diacetoxy-6-carboxy-anthraquinone (IV) (300 mg) (obtained in Example C) was added to a mixture of potassium tert. butoxide (160 mg), hydroquinone (186 mg) and water 5,2 ml). This mixture was sonicated for 30 mins. and then introduced to an ampoule from which the air was removed with a stream of argon. The ampoule was sealed and left at 130°C for 21 days. The ampoule was cooled, opened and its content poured into water. The resulting material was extracted with ethyl acetate. The organic extract was evaporated to dryness and the residue was chromatographed on a silica gel column. The material eluted with ethyl acetate: methanol (1:1) consisted of 30 mg of 1,3,4,6-tetrahydroxy-10,13-dicarboxy-helianthrone (I,R=COOH).
NMR ( CD₃COCD₃): 6,53 (2H s 2,5-H), 8.05 (2H, d J=7Hz 8,15-H), 8.40 (2H d J=9Hz 8,15-H), 8.49 (d J=9Hz 11,12-H).

### Example E

The diacid obtained in Example D (20 mg) was treated with absolute MeOH (10 ml) containing 3 drops of sulfuric acid and refluxed for 24 hours. The resulting solution was washed with a saturated solution of sodium bicarbonate and water. Evaporation to dryness resulted in a residue which. was chromatographed on silica gel. Elution with ethyl acetate: methanol (4:1) gave 11 mg of 1,3,4,6-tetrahydroxy-10,13-di(methoxy-carbonyl)-helianthrone (I,R=COOCH₃).
UV-vis (EtOH) λ ₘₐₓ371, 496, 550 sh, 580 nm (ε 27000, 32000, 25000, 26000).
NMR δ (CD₃COCD₃): 3.69 (6H s COOCH₃), 6,38 (2H s 2-H, 5-H), 8.05 (2H d J=2Hz 8,15-H), 8.47 (2H s 11,12-H), 8.59 (2H d J=2Hz 9,14-H).

### Example F

1,3,4,6-tetrahydroxy-helianthrone (30 mg) obtained as in Example A was dissolved in 10 ml acetone, treated with 1 g potassium carbonate, 0.6 ml dimethyl sulphate and refluxed for 24 hrs. The reaction mixture was then stirred for 30 min. with 20 ml. water and then extracted with 50 ml ethyl acetate. The organic extract was dried with magnesium sulphate, filtered and evaporated in vacuum to dryness, to give 28 mg. of 1,3,4,6-tetrametoxy-helianthrone.
UV-vis (EtOH) λₘₐₓ 458, 348, 331 nm
NMR δ (CDCl₃)3.96 (6H s 3,4-OCH₃) 4.15 (3H s 1-CH₃) 4.24 (3H s 6-CH₃) 6.91 (2H s 2,5-H) 725 (2H,t,d J = 12.5,1.5Hz, 9,14-H)7.45 (2H,t,d J=12,1.4, 10,13-H) 7.65 (2H,d,J=8Hz, 7,12-H) 8.28 (2H, d J = 8Hz, 8,11-H)

### Example G

The 10,13-dimethyl-1,3,4,6-tetrahydroxy-helianthrone (50 mg) obtained in Example A was dissolved in 20 ml acetone, treated with 1g potassium carbonate, 1 ml dimethyl sulphate and refluxed for 24 hrs. The reaction mixture was stirred for 30 min. with 30 ml water and then extracted with 50 ml ethyl acetate. The organic extract was dried with magnesium sulphate, filtered and evaporated in vacuum to dryness, to give 40 mg of 10,13-dimethyl-1,3,4,6-tetrametoxy-helianthrone.
UV-vis (EtOH)λₘₐₓ 458,348,331 nm
NMRδ (CDCI₃) 2.13 (6 H s 10, 13-CH₃) 3.96 (6 H s 3,4-OCH₃) 4.13 (3H s 1-CH₃) 4.21 (3H s 6-CH₃) 6.88(2H s 2,5-H) 7.254 (2H,q J = 10 Hz,3Hz) 7.55 (2H s 10,13-H) 8.28 (2H,d J = 8 Hz).

### BIOLOGICAL EXAMPLES

### Experimental Procedures

### A. Cell lines:

HL-60 leukemic cells were grown in RPMI-1640 supplemented with 15% fetal calf serum, 100 mM glutamine and 100 units/ml penicillin-streptomycin. K-562 cells were grown in the same medium supplemented with 10% fetal calf serum. Both cell lines were cultured in a humidified 5% CO₂/95% air atmosphere at 37°C.

### B. Cell viability:

Cell viability was monitored by the MTT assay which measures formation of formasan from 3-(4,5-dimethylthiazol-2-yl)-2,5-diphenyl tetrazolium bromide by viable cells as described in Mossman, T., *J. Immunogen*., **21**, 235-248 (1983).

### C. Photodynamic stress:

Photodynamic (PD) stress is the level of phototoxicity inflicted upon target cells by photodynamic compounds and exposure to light. Light irradiation was performed from a fluorescent source of two parallel 40 Watts tubes placed at a fixed distance of 16 cm and measured to emit an incidence of 4 mWatt/cm². Light intensities were quantitated using the IL 1350 Radiometer/Photometer, from International Light Inc., U.S.A.

### D. Determination of percentage of apoptotic cells:

Percentage of apoptotic cells was determined by light microscopy on cytospin cell preparations stained with May-Grunwald-Giemsa. 400 cells were counted by two individuals, independently, and the data are given as the average of the counts. Apoptotic cells were recognized by their smaller size and nuclei that were fragmented into condensed chromatin bodies.

### F. Flow cytometry analysis:

Cells harvested 5 hours after application of photodynamic stress were rinsed with phosphate buffered saline (PBS) and fixed with 70% aqueous ethanol. The cells were then resuspended in phosphate-citrate buffer (PC buffer) pH 7.8 (192 parts of 0.2 M Na₂PHO₄ and 8 parts of 0.1 M citric acid) at room temperature for 30 minutes and stained with propidium iodide in PC buffer containing 10 µg/ml RNase A. The cells were then analyzed in a Coulter EPICS XL-MCL flow cytometer with the entire field gated to include the various changes that affected the cells.

### F. DNA fragmentation Assay:

DNA fragmentation in cells undergoing apoptosis was assayed as described previously (Lotem, J. and Sachs, L., *Cell Growth and Differ*., **6**, 647-653 (1995). 2×10⁶ cells pelleted in Eppendorf tubes were lysed in 0.5 ml lysis buffer containing 10 mM Tris-HCI, pH 7.5, 0.6% SDS, 10 mM EDTA and 15 µg/ml RNA mixture (Ambion Corp., Austin TX). After incubation at 37°C for 10 minutes, NaCl was added to 1 M and the mixture was kept overnight at 4°C. The preparation was spun at 14,000 g for 30 minutes at 4°C, the supernatant collected, phenol extracted and DNA precipitated overnight at -20°C by adding 1 ml ethanol.

The DNA pellet was air-dried, dissolved in 20 µl TE buffer (10 mM Tris, 10 mM EDTA, pH 7.5) at 4°C for 24 hours, electrophoresed for 4 hours at 2V/cm in 1.5% agarose gel containing 0.5 µg/ml ethidium bromide and photographed under U.V. light.

### Example 1 The photodynamic effects of HY and DTHe on HL-60 cell viability

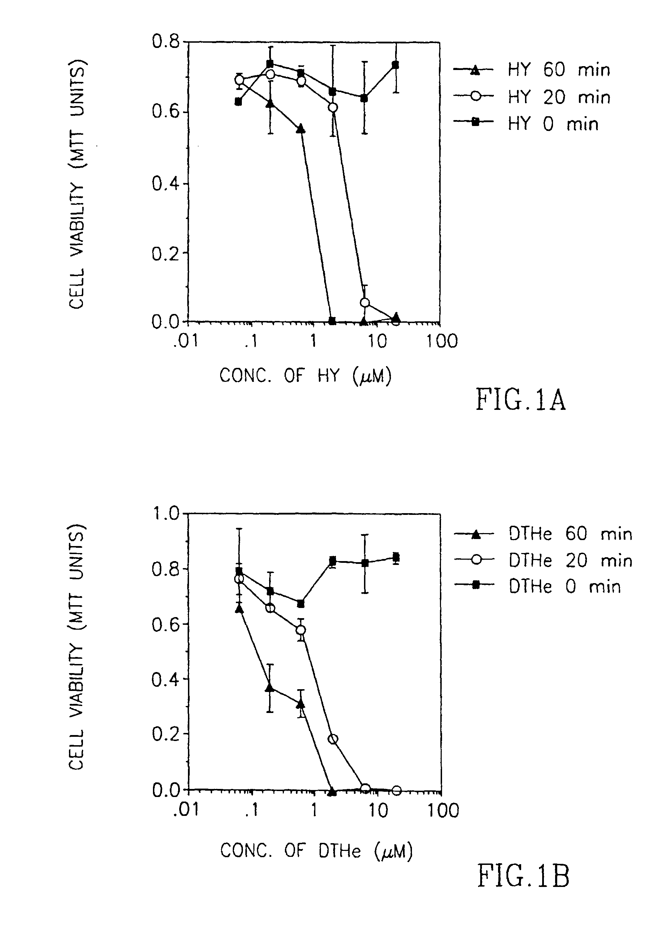

The phototoxicity of HY and DTHe to HL-60 cells, as a function of the applied photodynamic stress, was compared following exposure to two doses of light irradiation: 4.8 or 14.4 Joule/cm², obtained by irradiation for 20 or 60 minutes, respectively. Cells were plated in duplicate 10⁵ cells/well in 100 µl of medium in 96 well microplates. HY and DTHe were added at 2x concentrations to yield 200 µl in final concentrations that range from 0.66-20 µM with 0.5 log₁₀ dose increments. Irradiation was carried out from a fluorescent source at an intensity of 4 mWatts/cm² for 0, 20 (4.8 Joule/cm²) and 60 minutes (14.4 Joule/cm²). Cell viability was monitored after 16 hours by the MTT assay. The results, shown in Fig. 1, indicate that DTHe exhibited a more potent phototoxic activity in comparison with HY. Cell death with DTHe occurred with an LD₅₀ of 1 µM at 4.8 Joule/cm², which is about 3-fold lower than that of HY (3 µM). A more potent phototoxic activity of DTHe was also seen at the higher light dose of 14.4 Joule/cm² (LD₅₀ of 0.15 µM and 0.7 µM for DTHe and HY, respectively). In DTHe and in HY treated cells, cell viability, thus, declined in a dose-dependent manner of both light incidence and concentration of the compounds (Figs. 1A and 1B). There was no loss of cell viability when the treatments with DTHe or HY were conducted in the absence of light for the same time length, or when the cells were exposed to light in the absence of the compounds. The results, therefore, indicate that cell death resulted from photodynamic effects.

### Example 2

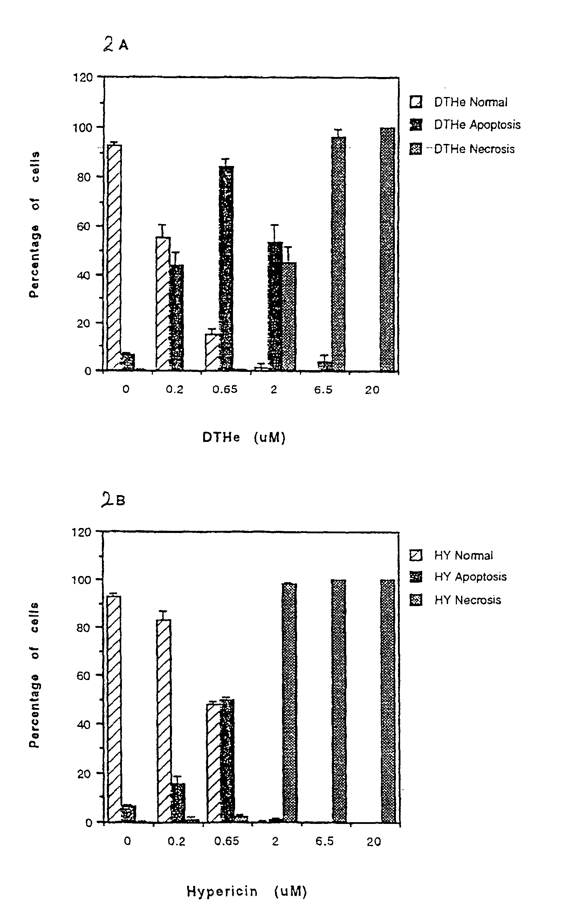

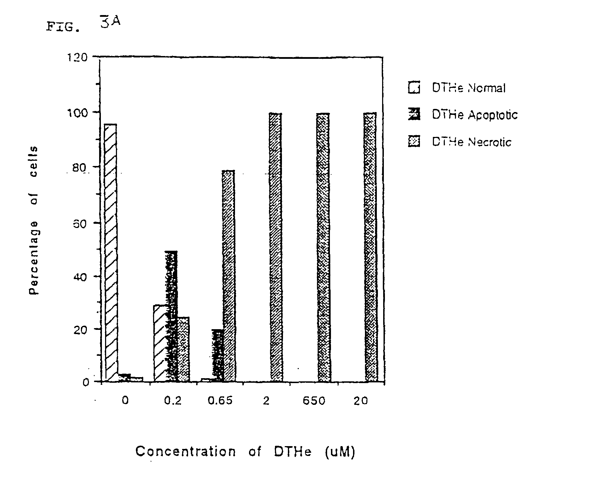

The modes of cell death induced by the photodynamic effects of HY and DTHe were evaluated by comparative microscopy of photosensitized HL-60 cytospin cell preparations. Cells were exposed to HY or to DTHe at concentrations that ranged from 0.65-20 µM (0.5 log₁₀ increments) and light irradiation at 7.2 Joule/cm² and cultured for 5 hours. Cytospin preparations were then prepared from 50% of the cells and stained with May-Grunwald-Giemsa. 500 cells were then counted in each preparation. The remaining 50% of the cells were washed with PBS, fixed with 70% EtOH, stained with propidium iodide and analyzed by flow cytometry (4C). Lane 1 (left panel) untreated cells; lanes 2-5 HL-60 cells exposed to 0.1, 0.65, 1 and 6.5 µM DTHe, respectively. Normal, apoptotic and necrotic cells were scored, and the quantitative results are shown in Fig. 2. At the lower dose range of 0.2-2.0 µM DTHe, the prevalent mode of cell death was apoptosis (Fig. 2A). At 0.65 µM DTHe apoptosis was the only form of cell death recognizable. However, as doses of DTHe were increased to 6.5-20.00 µM, cell death occurred via a peculiar form of apparent necrosis. Photoinduced "*necrosis*" was associated with enlargement of the nuclei and formation of a characteristic perinucleolar, ring-like condensations of chromatin that were resistant to further increases in photodynamic damage (data not shown). With hypericin (HY), formation of apoptotic bodies occurred at ≤ 2 µM, but at doses ≥ 2µM cell death was apparently mainly necrotic (Fig. 2B). These findings indicate that, although HY was a less potent photoinducer of cell death, HY-induced necrosis occurred at concentrations that were 3-fold lower than DTHe doses that caused apparent necrosis.

### Example 3

To further characterize the nature of the degraded DNA in DTHe treated HL-60 cells, the pattern of DNA digestion by electrophoresis in agarose gels was examined. The patterns were correlated with the morphology of the cells as visualized from cytospin stained preparations assayed for percentage of apoptotic and necrotic cells. The results, suggest that PD stress induced by DTHe and HY caused cell death via an apoptotic mechanism. The apparent necrotic morphology obtained at high PD stress levels resulted from impairment of some elements in the cellular machinery that fragments the cells into discrete apoptotic bodies. The endonuclease appeared to be more resistant to the photo-oxidative damage than the nuclear disintegration process.

### Example 4 Effects on K-562 cells

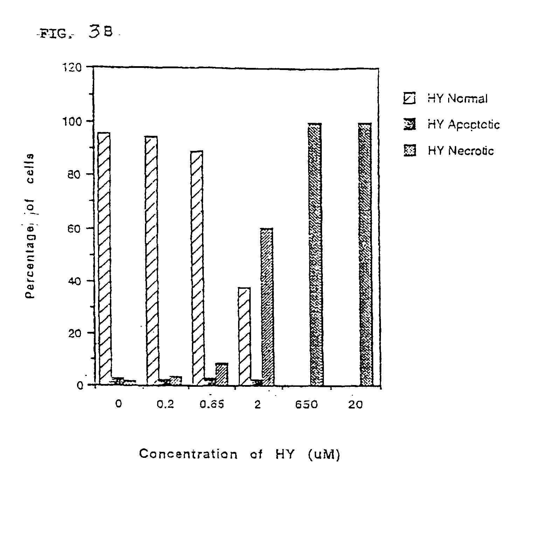

Photodynamic stress was also applied to K-562 cells with DTHe and with HY and its effect on cell death was analyzed microscopically. K-562 cells were exposed to DTHe (3A) and to HY (3B) with light (6.4 Joule/cm²) and were cultured for 6 hours. Cytospin preparations were then prepared, stained with May-Grunwald Giemsa and counted for normal viable cells, apoptotic bodies and necrotic cells. The results, shown in Fig. 3 indicate that, although the sensitivity of K-562 cells to DTHe or HY-mediated phototoxicity was similar to that of HL-60 cells, K-562 cells appeared less prone to undergo complete photodynamic-induced apoptosis with apoptotic body morphology than HL-60 cells. With HY as the photoactivator almost no apoptotic figures were detected at any of the doses applied and apparently necrotic cells were induced at ≥ 2 µM. DNA fragmentation to oligonucleosomes in K-562 cells also occurred at the higher doses of 2 ≥ µM and 7.2 Joule/cm² light irradiation, and the dose range for cells with apoptotic morphology (0.2-0.65 µM (Fig. 3A) was narrower than the apoptotic range seen in HL-60 cells (Fig. 2A).

### Example 5 THe as a photodynamic agent

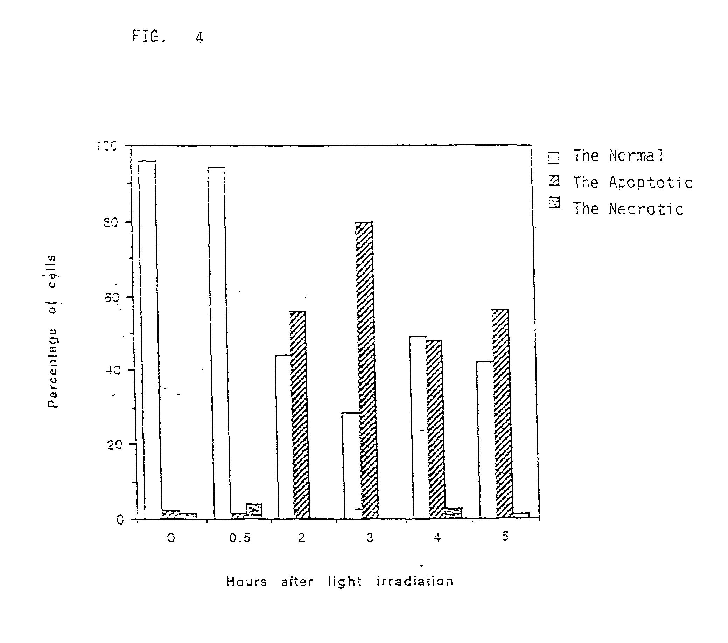

HL-60 cells 4×10⁵ ml in medium RPMI-1640 supplemented with 15% fetal calf serum, received 1,3,4,6-tetrahydroxy-helianthrone (Wis-2) (THe) at a concentration of 0.65 µM. The cells were light irradiated with 14.4 Joule/cm² and incubated in a 37°C incubator. Samples were then collected 0.5 hours, 1, 2, 3, 4 and 5 hours after light irradiation, concentrated on a glass slide by cytospin, stained in May-Grunwald-Giemsa and scored by microscopy for normal, apoptotic and necrotic cells. The results are shown in Fig.4. Again apoptosis was the predominant mechanism of cell death induction which peaked 3 hours after light irradiation.

### Example 6

### Protection of L-cells from TNF -α by DTHe

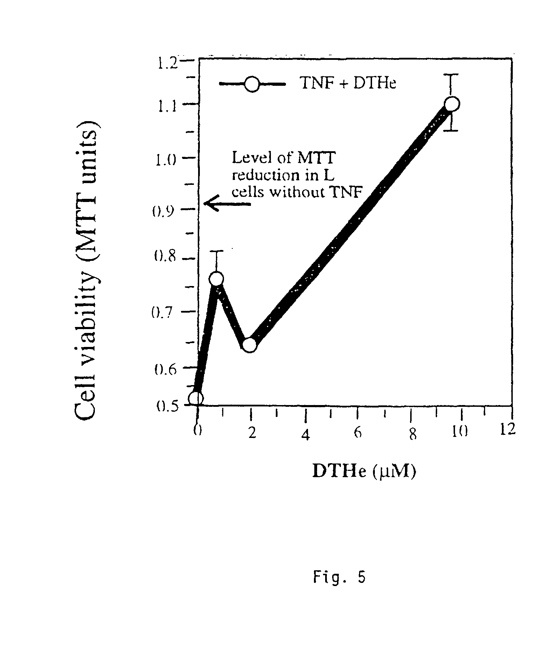

The effect of DTHe on TNF-α induced apoptosis (programmed cell death) in murine cells was determined: L-cells were placed in 96 well plates at a concentration of 5 × 10⁴ cells per well in medium RPMI-1640 supplemented with 10%-serum, 10µml penicillin and 10µml streptomycin. After 12 hours, DTHe was administered to the cells at doses of 0.5, 2 and 10µM followed by TNF-α 100µM and the cells were cultured for an additional 24 hours at 37°C (5%CO₂). An MTT assay was then performed to assess the cell viability (Mossman, T. A rapid colorimetric assay for cellular growth and survival: application to proliferation and cytotoxic assays, *J. Immunogen*, *21,p. 235, 1983).* The results are shown in Fig. 5. Data are averages of triplicate wells ± S.D. from one representative experiment of three performed with this cell line.

### Example 7

### Effect of DTHe on NK cell cytotoxic activity against K-562 cells

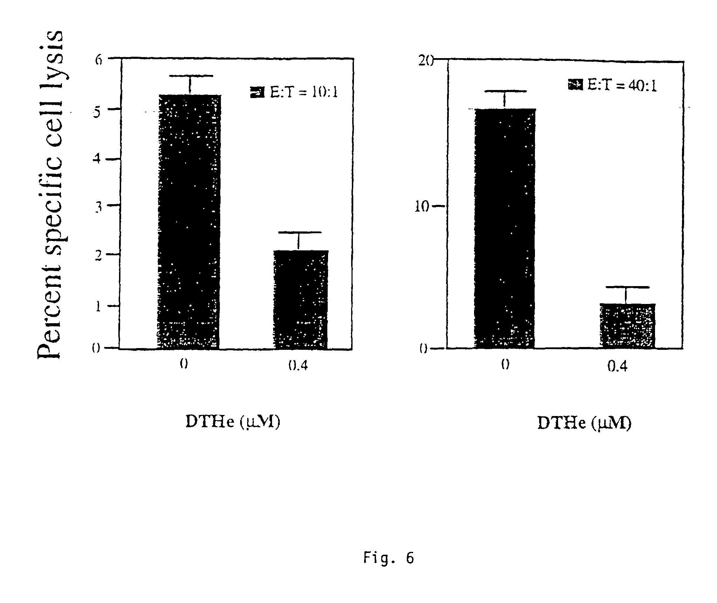

Effect of DTHe on human natural killer (NK) cell mediated cytotoxicity was established by determining the cytotoxic activity of NK cells on K-562 target cells labeled with radioactive chromium (Na₂⁵¹CrO₄). The target cells were then brought into contact with NK cells isolated from human peripheral blood, and their cytotoxic activity against the K-562 targets was established by measuring the amount of radioactive chromium released from the damaged K-562. The experiment was conducted bringing into contact effector NK-cells (E) and K-562 target cells (T at 2 effector target cell ratios E:T = 40:1 and E:T = 10:1). The results are shown in Fig. 6.

### LITERATURE REFERENCES

1. Dougherty, T.J., Hematoporphyrin as a photosensitizer of tumors, *Photochemistry and Photobiology*, **38**:377-379, 1983.
2. Honigsmann, H., Tanew, A., and Wolff, K., Treatment of mycosis fungoides with PVVA. *Photo-Dermatology*, **4**:55-58, 1987.
3. Thomas, C., MacGill, R.S., Miller, G.C., and Pardini, R., *Photochem. and Photobiol*., **55**:47-S3, 1992.
4. Hadjur, C., Jeunet, A., and Jardon, P., *J. of Photochem. and Photobiol. B. Biol*., **26**:67-74, 1994.
5. Diwu, Z., Lown, J.W., *Free Radical Biol. Med*., **14**:209-215, 1993.
6. Lavie, G., Mazur, Y., Lavie D., and Mevuelo, D. The chemical and biological properties of hypericism- a compound with a braod spectrum of biological activities. *Medical Research Reviews*, **15**:111-119, 1994.
7. Agostinis, P., Vandenbogaerde, A., Donella-Deana, A., Pinna, L.A., Lee, K.T., Goris, J., Merlevede, W., Vandenheede, J.R., and De Witte, P. Photosensitized inhibition of growth factor regulated protein kinases of hypericin. *Biochemical Pharmacology*, **49**:1615-1622, 1995.

## Claims

1. Use of compounds of the general formula (I) 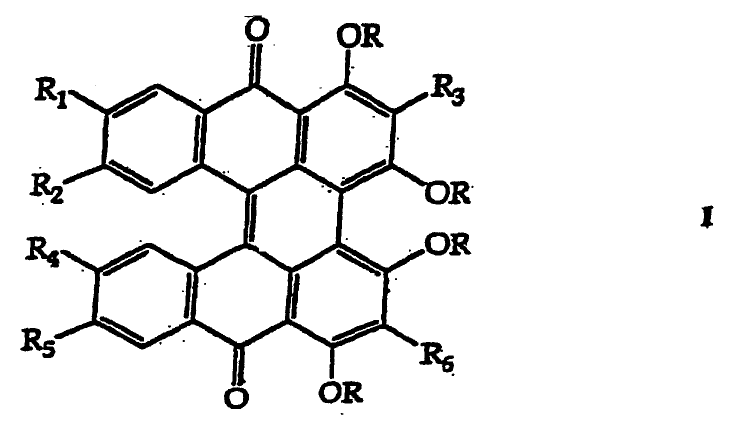 in which R is hydrogen or methyl, each of R₁, R₂, R₃, R₄, R₅ and R₆ is independently selected from H, OH, Cl, Br, methyl and methoxycarbonyl in the manufacture of pharmaceutical compositions for use in photodynamic therapy of tumors, to elicit destruction of tumors in conjunction with light

2. The use according to Claim 1 of the compounds of formula (I), in which R is hydrogen, R₂ and R₄ are the same and are each methyl or methoxycarbonyl and R₁, R₃, R₅ and R₆ are each H.

3. The use according to Claim 1, wherein the compound is 10,13-dimethyl-1,3,4,6-tetrahydroxy-helianthrone.

4. The use according to Claim 1, wherein the compound is 10,13-di(methoxycarbonyl)-1,3,4,6-tetrahydroxy-helianthrone.

5. The use according to Claim 1, wherein the compound is 1,3,4,6-tetrahydroxy-helianthrone.

6. The use according to claim 1, wherein the compound is 1,3,4,6-tetramethoxy-helianthrone.

7. The use according to claim 1, wherein the compound is 10,13-dimethyl-1,3,4,6-tetramethoxy-helianthrone.

8. Compounds of formula (I): 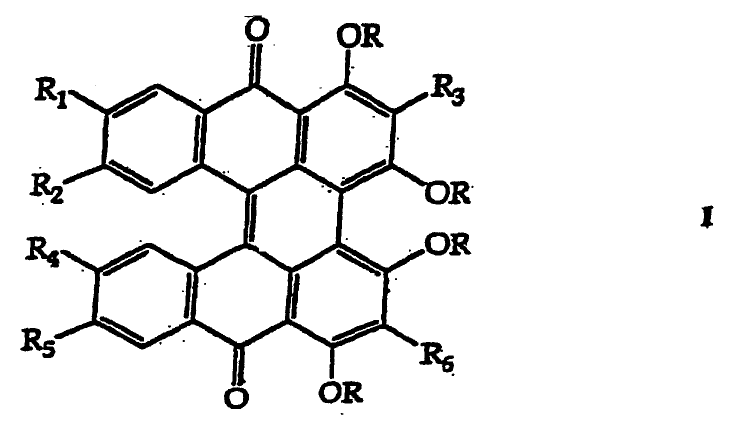 in which R is hydrogen or methyl, each of R₁, R₂, R₃, R₄, R₅ and R₆ is independently selected from H, OH, Cl, Br, methyl and methoxycarbonyl, wherein at least one of R and R₁ to R₆ is other than hydrogen.

9. The compounds according to claim 8, wherein R₂ and R₄ are the same and each methyl or methoxycarbonyl and R₁, R₃, R₅ and R₆ are each H.

10. 10,13-dimethyl-1,3,4,6-tetrahydroxy-helianthrone, according to claim 8.

11. 10,13-di(methoxycarbonyl)-1,3,4,6-tetrahydroxy-helianthrone, according to claim 8.

12. 1,3,4,6-tetramethoxy-helianthrone, according to claim 8.

13. 10,13-dimethyl-1,3,4,6-tetramethoxy-helianthrone, according to claim 8.

14. Pharmaceutical composition comprising a compound of formula (I) 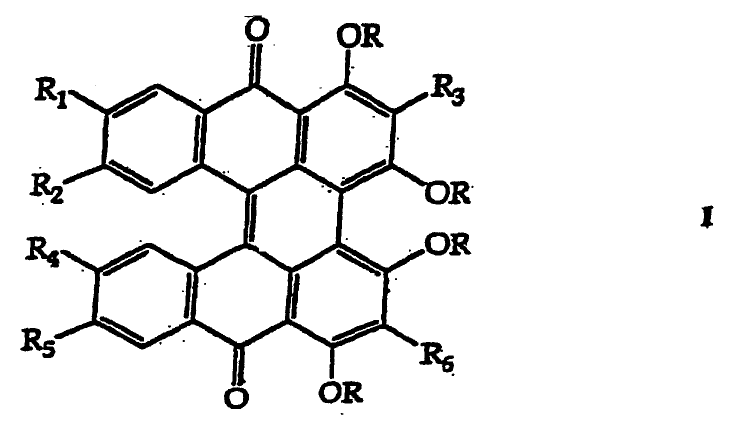 in which R is hydrogen or methyl, each of R₁, R₂, R₃, R₄, R₅ and R₆ is independently selected from H, OH, Cl, Br, methyl and methoxycarbonyl, wherein at least one of R and R₁ to R₆ is other than hydrogen together with pharmaceutically acceptable carriers.

15. Pharmaceutical compositions according to claim 14, wherein the carriers are acceptable for intravenous direct injection or topical application.

## Patentansprüche

1. Verwendung von Verbindungen mit der allgemeinen Formel (I) 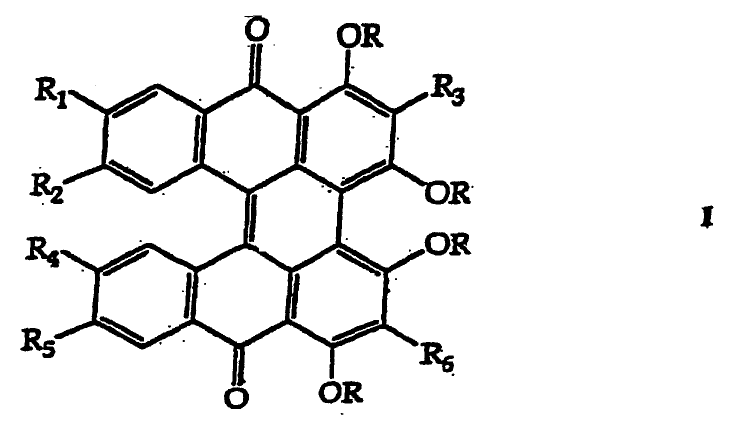 in welcher R Wasserstoff oder Methyl ist, R₁, R₂, R₃, R₄, R₅ und R₆ jeweils unabhängig voneinander ausgewählt sind aus H, OH, Cl, Br, Methyl und Methoxycarbonyl, bei der Herstellung von pharmazeutischen Zusammensetzungen zur Anwendung in der photodynamischen Therapie von Tumoren, um eine Zerstörung der Tumoren in Verbindung mit Licht auszulösen.

2. Die Verwendung gemäß Anspruch 1 der Verbindungen der Formel (I), in welcher R Wasserstoff ist, R₂ und R₄ gleich sind und jeweils Methyl oder Methoxycarbonyl sind und R₁, R₃, R₅ und R₆ jeweils H sind.

3. Die Verwendung gemäß Anspruch 1, wobei die Verbindung 10,13-Dimethyl-1,3,4,6-tetrahydroxyhetianthron ist.

4. Die Verwendung gemäß Anspruch 1, wobei die Verbindung 10,13-Di(methoxycarbonyl)-1,3,4,6-tetrahydroxyhelianthron ist.

5. Die Verwendung gemäß Anspruch 1, wobei die Verbindung 1,3,4,6-Tetrahydroxyhelianthron ist.

6. Die Verwendung gemäß Anspruch 1, wobei die Verbindung 1,3,4,6-Tetramethoxyhelianthron ist.

7. Die Verwendung gemäß Anspruch 1, wobei die Verbindung 10,13-Dimethyl-1,3,4,6-tetramethoxyhelianthron ist.

8. Verbindungen der Formel (I): 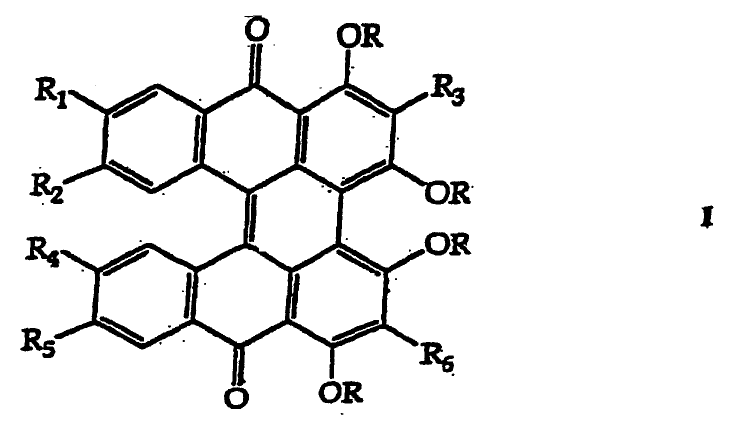 in welcher R Wasserstoff oder Methyl ist, R₁, R₂, R₃, R₄, R₅ und R₆ jeweils unabhängig voneinander ausgewählt sind aus H, OH, Cl, Br, Methyl und Methoxycarbonyl, wobei wenigstens einer von R und R₁ bis R₆ von Wasserstoff verschieden ist.

9. Die Verbindungen gemäß Anspruch 8, wobei R₂ und R₄ gleich sind und jeweils Methyl oder Methoxycarbonyl sind und R₁, R₃, R₅ und R₆ jeweils H sind.

10. 10,13-Dimethyl-1,3,4,6-tetrahydroxyhelianthron gemäß Anspruch 8.

11. 10,13-Di(methoxycarbonyl)-1,3,4,6-tetrahydroxyhelianthron gemäß Anspruch 8.

12. 1,3,4,6-Tetramethoxyhelianthron gemäß Anspruch 8.

13. 10,13-Dimethyl-1,3,4,6-tetramethoxyhelianthron gemäß Anspruch 8.

14. Pharmazeutische Zusammensetzung, umfassend eine Verbindung der Formel (I) 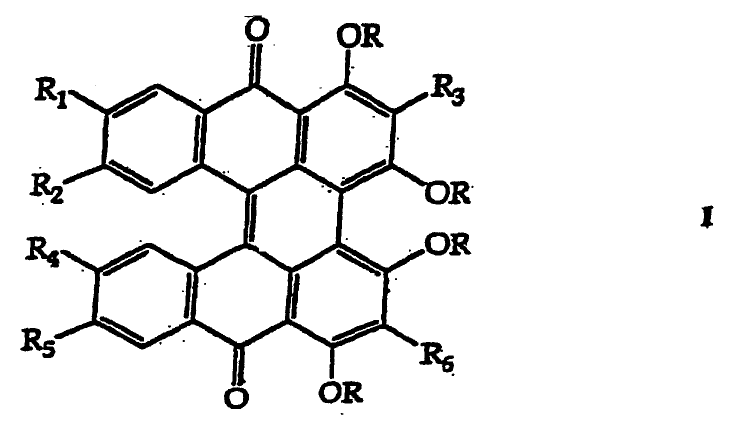 in welcher R Wasserstoff oder Methyl ist, R₁, R₂, R₃, R₄, R₅ und R₆ jeweils unabhängig voneinander ausgewählt sind aus H, OH, Cl, Br, Methyl und Methoxycarbonyl, wobei wenigstens einer von R und R₁ bis R₆ von Wasserstoff verschieden ist, zusammen mit pharmazeutisch verträglichen Trägern.

15. Pharmazeutische Zusammensetzungen gemäß Anspruch 14, wobei die Träger zur intravenösen direkten Injektion oder zur topischen Anwendung geeignet sind.

## Revendications

1. Utilisation de composés de formule générale (I) 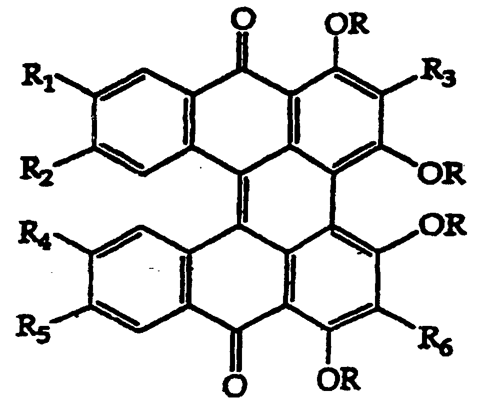 dans laquelle R est un atome d'hydrogène ou un groupe méthyle, chacun des radicaux R₁, R₂, R₃, R₄, R₅ et R₆ est choisi indépendamment parmi H, OH, Cl, Br, méthyle et méthoxycarbonyle, dans la fabrication de compositions pharmaceutiques destinées à une utilisation en thérapie photodynamique de tumeurs, pour entraîner la destruction de tumeurs en conjonction avec la lumière.

2. Utilisation selon la revendication 1 des composés de formule (I), dans laquelle R est un atome d'hydrogène, R₂ et R₄ sont identiques et sont chacun un groupe méthyle ou méthoxycarbonyle et R₁, R₃, R₅ et R₆ sont chacun H.

3. Utilisation selon la revendication 1, dans laquelle le composé est la 10,13-diméthyl-1,3,4,6-tétrahydroxy-hélianthrone.

4. Utilisation selon la revendication 1, dans laquelle le composé est la 10,13-di(méthoxycarbonyl)-1,3,4,6-tétrahydroxy-hélianthrone.

5. Utilisation selon la revendication 1, dans laquelle le composé est la 1,3,4,6-tétrahydroxy-hélianthrone.

6. Utilisation selon la revendication 1, dans laquelle le composé est la 1,3,4,6-tétraméthoxy-hélianthrone.

7. Utilisation selon la revendication 1, dans laquelle le composé est la 10,13-diméthyl-1,3,4,6-tétraméthoxy-hélianthrone.

8. Composés de formule générale (I) 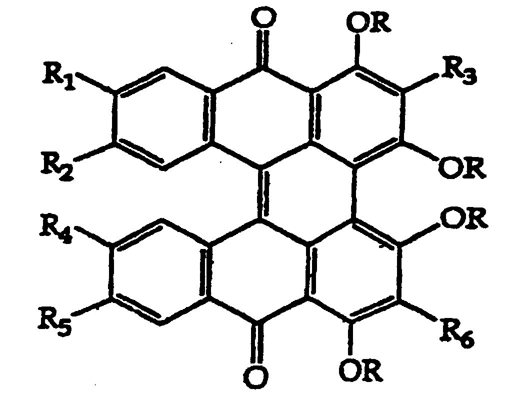 dans laquelle R est un atome d'hydrogène ou un groupe méthyle, chacun des radicaux R₁, R₂, R₃, R₄, R₅ et R₆ est choisi indépendamment parmi H, OH, Cl, Br, méthyle et méthoxycarbonyle, dans laquelle l'un au moins des radicaux R et R₁ à R₆ est autre qu'un atome d'hydrogène.

9. Composés selon la revendication 8, dans laquelle R₂ et R₄ sont identiques et sont chacun un groupe méthyle ou méthoxycarbonyle et R₁, R₃, R₅ et R₆ sont chacun H.

10. 10,13-diméthyl-1,3,4,6-tétrahydroxy-hélianthrone selon la revendication 8.

11. 10,13-di(méthoxycarbonyl)-1,3,4,6-tétrahydroxy-hélianthrone selon la revendication 8.

12. 1,3,4,6-tétraméthoxy-hélianthrone selon la revendication 8.

13. 10,13-diméthyl-1,3,4,6-tétraméthoxy-hélianthrone selon la revendication 8.

14. Compositions pharmaceutiques comprenant un composé de formule générale (I) 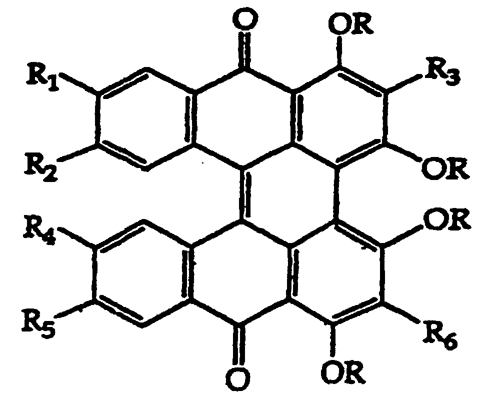 dans laquelle R est un atome d'hydrogène ou un groupe méthyle, chacun des radicaux R₁, R₂, R₃, R₄, R₅ et R₆ est choisi indépendamment parmi H, OH, Cl, Br, méthyle et méthoxycarbonyle, dans laquelle l'un au moins des radicaux R et R₁ à R₆ est autre qu'un atome d'hydrogène, avec des véhicules acceptables au plan pharmaceutique.

15. Compositions pharmaceutiques selon la revendication 14, dans lesquelles les véhicules sont acceptables pour une injection intraveineuse directe ou une application locale.
